# EUROPEAN PATENT SPECIFICATION

(11) **EP 2 019 638 B1**
(45) Date of publication and mention of the grant of the patent: **01.12.2010**
(21) Application number: 07761313.1
(22) Date of filing: 26.04.2007
(51) Int. Cl.: A61B 17/70, A61B 17/86

(54) **METHOD OF MANUFACTURING A BONE ANCHOR SYSTEM UTILIZING A MOLDED COUPLING MEMBER FOR COUPLING A BONE ANCHOR TO A STABILIZATION MEMBER**
VERFAHREN ZUR HERSTELLUNG EINES KNOCHENANKERSYSTEMS MIT EINEM GEFORMTEN KOPPLUNGSELEMENT ZUR KOPPLUNG EINES KNOCHENANKERS AN EIN STABILISIERUNGSELEMENT
MÉTHODE DE FABRICATION D'UN SYSTÈME D'ANCRAGE OSSEUX UTILISANT UN ÉLÉMENT DE COUPLAGE MOULÉ POUR COUPLER UN ANCRAGE OSSEUX À UN ÉLÉMENT STABILISANT

(30) Priority: 01.05.2006 US 414878
(43) Date of publication of application: 04.02.2009
(73) Proprietor: Warsaw Orthopedic, Inc., Warsaw, Indiana 46581 (US)
(72) Inventor: DEWEY, Jonathan, M., Sunnyvale CA 94085 (US); PATTERSON, Christopher, M., Olive Branch, Mississippi 38654 (US)
(74) Representative: Hutchinson, Glenn Stanley
(86) International application number: PCT/US2007/067455
(87) International publication number: WO 2007/130835

(56) References cited:
- WO-A-97/02786
- WO-A-98/32386
- FR-A1- 2 796 545
- US-A1- 2001 047 173
- US-A1- 2004 053 196
- US-A1- 2004 225 289

## Description

### Technical Field

The present invention relates generally to surgical implants for bone and skeletal stabilization and, more particularly, to a bone anchor system for coupling a bone anchor to a stabilization member.

### Background of the Invention

In the field of spinal surgery, for example, it is customary to place implants into vertebrae for a number of reasons, including: (a) correcting an abnormal curvature of the spine; (b) to maintain appropriate vertebral spacing and provide support for broken or otherwise injured vertebrae; and (c) to perform other treatments in the spinal column. In other orthopedic applications, implants are used to support and maintain proper alignment of broken bones while they mend.

Typical spinal implant or other bone stabilization systems utilize a rod as a support and stabilizing element. In such a spinal implant system, a series of two or more bone fasteners are inserted into two or more vertebrae to be supported. A rod or other stabilizing member is then placed within or attached to the heads of the bone fasteners. Alternatively, a stabilizing member is placed within a coupling device that links the stabilizing member and the head of the bone fastener. The connections between these multiple components are then secured, thereby fixing a supporting construct to multiple levels in the spinal column.

In some orthopedic applications, it would be advantageous from a medical standpoint to use a stabilization member that is less rigid than a rod made of a biocompatible metal such as titanium or stainless steel. It has been determined that the use of a conventional coupling member made of biocompatible metal can result in undesirable stress concentrations at various points on the surface of stabilization members made of more flexible materials when in vivo post-operative loads are applied to the stabilization system.

Therefore, there is a need for a bone stabilization system that reduces the occurrence of localized stress concentrations in stabilization members made of materials that are more flexible than conventional biocompatible metals. Also, because conventional coupling devices are assembled from a number of separate parts, it would be advantageous to provide a bone anchor system having fewer parts to assemble.

FR 2796545 A1 discloses a polyaxial connector for spinal support frame. WO 98/32386 A relates to a device for connecting a longitudinal bar to a pedicle screw which forms part of a system for fixing the spinal column. US 2004/053196 A1 describes an implant to be implanted in bone tissue. US 2004/225289 A1 describes a dynamic anchoring device. US 2001/047173 A1 relates to a device for connecting a longitudinal support to a bone anchor having a rounded head. WO 97/02786 A discloses a polyaxial colletted locking mechanism for use with orthopaedic apparatus.

### Summary of the Invention

The present invention relates to a method of manufacturing a bone anchor system as claimed hereafter. Preferred methods of the invention are set forth in the dependent claims.

The shortcomings of the prior art are overcome and additional advantages are provided, in one aspect, through a bone anchor system comprising a bone anchor having an integral head, a stabilization member, and a coupling member molded around the integral head of the bone anchor for coupling the bone anchor to the stabilization member.

In another aspect, a bone anchor system comprises a bone anchor having an integral head, a stabilization member, and a coupling member for coupling the bone anchor to the stabilization member, wherein the coupling member comprises an interface to the stabilization member, and the interface comprises a deformable material.

In a further aspect, a bone anchor system, in accordance with the present invention comprises: a stabilization member; a bone anchor having an integral head, which comprises a molded cap configured to provide an interface to the stabilization member, wherein the molded cap comprises a deformable material; and a coupling member for coupling the bone anchor to the stabilization member.

Yet another aspect of the invention is a method of providing a coupling between a stabilization member and bone anchor of a bone anchor system in which the method comprises: (i) molding a coupling member to an integral head of a bone anchor; (ii) providing, in the coupling member, an interface to a stabilization member; (iii) providing, in the coupling member, a means for engaging a retaining member; and (iv) configuring the coupling and the retaining members to cooperatively retain the stabilization member when the stabilization member is placed in the interface of the coupling and the retaining member is engaged in the means for engaging the retaining member.

Further, additional features and advantages are realized through the construction and techniques of the present invention. Other embodiments and aspects of the invention are described in detail herein and are considered a part of the claimed invention.

### Brief Description of the Drawings

The subject matter which is regarded as the invention is particularly pointed out and distinctly claimed in the claims at the conclusion of the specification. The foregoing and other objects, features, and advantages of the invention are apparent from the following detailed description taken in conjunction with the accompanying drawings in which:
FIGS. 1A-1E illustrate several views of an embodiment of a bone anchor system in which the coupling member is molded to the head of a bone anchor in a fixed orientation, in accordance with an aspect of the present invention;
FIGS. 2A-2G illustrate several views of an embodiment of a bone anchor system in which the coupling member is molded to the head of a bone anchor and configured to pivot about the head of the bone anchor, in accordance with an aspect of the present invention;
FIGS. 3A-3I illustrate several views of an embodiment of a bone anchor system wherein a coupling member, which is molded to the head of a bone anchor, includes an inserted interface piece comprising a deformable material that provides an interface to a stabilization member, in accordance with an aspect of the present invention;
FIGS. 4A-4J illustrate several views of an embodiment of a bone anchor system wherein an inserted interface piece, which comprises a deformable material and provides an interface to a stabilization member, may slide along an arc determined by a pair of groves in a coupling member, which is molded to the head of a bone anchor, in accordance with an aspect of the present invention;
FIGS. 5A-5J illustrate several views of an embodiment of a bone anchor system in which the coupling member and inserted interface piece, which comprises a deformable material, are configured to arrest the pivoting of the coupling member about the head of a bone anchor if a retaining member engages the coupling member, in accordance with an aspect of the present invention;
FIG. 6 illustrates a cutaway view of an embodiment of a bone anchor system comprising a conventional coupling member that is adapted to accept an interface piece comprising a deformable material, in accordance with an aspect of the present invention;
FIG. 7 illustrates a cutaway view of an embodiment of a bone anchor system wherein a coupling member, which is moldcd to the head of a bone anchor, engages a threaded insert configured for engaging a retaining member, in accordance with an aspect of the present invention; and
FIGS. 8A and 8B illustrate two views of a bone anchor system wherein the integral head of a bone anchor comprises a molded cap of deformable material, and the molded cap provides an interface to a stabilization member, in accordance with an aspect of the present invention.

### Best Mode for Carrying Out the Invention

The present invention is directed to a bone anchor system comprising a bone anchor (e.g., a screw) having an integral head, a stabilization member (e.g. a rod), and a coupling member for coupling the bone anchor to the stabilization member. In one embodiment of a bone anchor system, in accordance with an aspect of the present invention, the coupling member is molded around the integral head of the bone anchor. In accordance with another aspect of the invention, the coupling member provides an interface to a stabilization member (e.g. a rod). The interface provided by the coupling member is less rigid than the bone anchor or metal couplings used in conventional implantable bone anchor systems. For example, the coupling member or at least the interface may be molded from a polyetheretherketone (PEEK) material, which is a high-strength plastic that is radiolucent, non-conducting, and non-magnetic.

The provision of a less-rigid, deformable interface to the stabilization member has the potential advantage of increasing the life of non-metal stabilization members because it is less likely that the stresses transmitted through such an interface will cause notches, abrasions, or discontinuities in the surface of a non-metal stabilization member. Another potential advantage of providing an interface comprising a deformable material is that the stabilization member may be more securely held in the coupling member. As one with ordinary skill in the art will appreciate, the interface to the stabilization member may comprise other biocompatible, deformable materials. Coupling members made from radiolucent, non-conducting, and non-magnetic materials such as polymers, carbon fiber materials, resins, nylon, and silicone arc advantageous because such materials are more compatible with common medical imaging techniques. As an example, coupling members may be molded from polyetheretherketone materials. A number of embodiments of the present invention are described in more detail below and illustrated in the drawings.

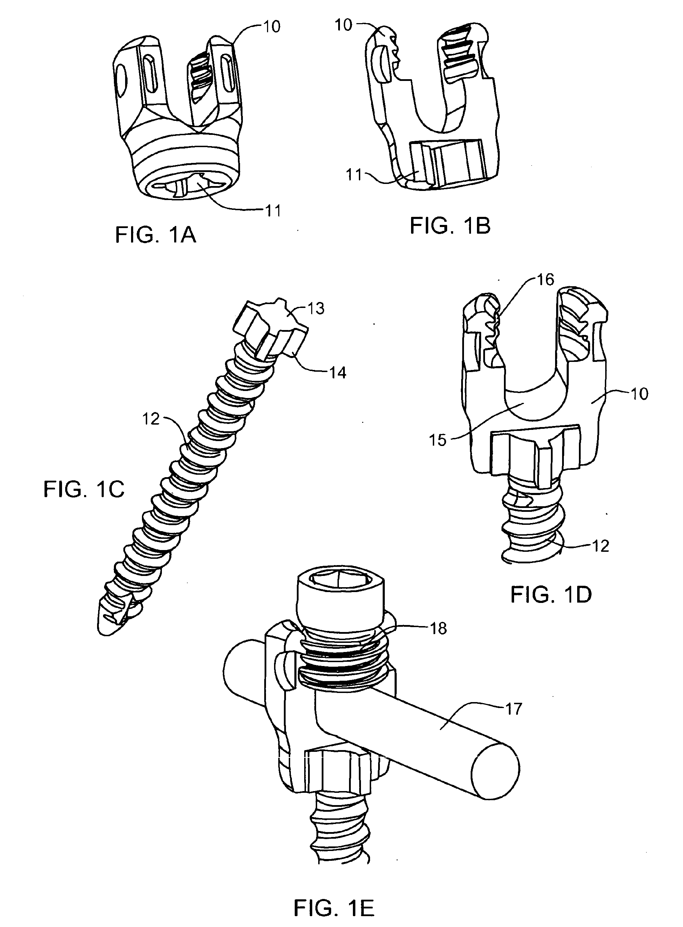

FIGS. 1A-1E illustrate several views of an embodiment of a bone anchor system in which the coupling member is molded to the head of a bone anchor in a fixed orientation, in accordance with an aspect of the present invention. In this embodiment, coupling member 10 is molded around integral head 13 of bone anchor 12. As illustrated in an exemplary embodiment of bone anchor 12 in FIG. 1C, integral head 13 comprises one or more features 14 that facilitate holding coupling member 10 to integral head 13 of bone anchor 12. FIG. 1A and the cutaway view of FIG. 1B illustrate two views of cavity 11 in coupling member 10 that is formed as a result of molding coupling member 10 to integral head 13 of bone anchor 12. FIG. 1D illustrates a cutaway view of coupling member 10 molded around the integral head of bone anchor 12. FIG. 1D also illustrates interface 15 of coupling member 10 that provides an interface to a stabilization member, such as a rod. In this embodiment, interface 15 to the stabilization member is integrally formed in molded coupling member 10. In addition, FIG. 1D illustrates threads 16 that arc formed in molded coupling member 10. FIG. 1E illustrates stabilization member 17 being held in coupling member 10 by retaining member 18. In the example shown in FIG. 1E, retaining member 18 comprises a setscrew that engages threads 16.

In embodiments in which the coupling member has an integrally formed interface to the stabilization member, the interface to the stabilization member preferably comprises a surface that is contoured to the shape of the stabilization member in the region of contact between the interface and stabilization member. For example, as illustrated in FIGS. 1A-1E, interface 15 to the stabilization member may comprise a U-shaped surface where the stabilization member has a cylindrical shape.

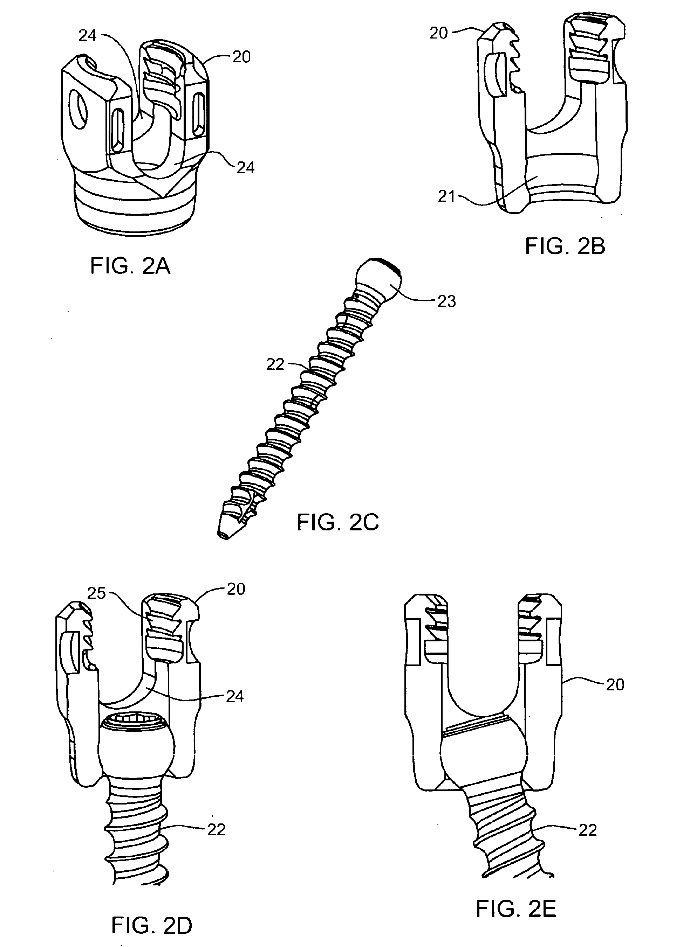

In another embodiment having a stabilization member interface integrally formed in a molded coupling member, the molded coupling member may pivot about a rounded or quasi-spherical head of a bone anchor, as illustrated in several views in FIGS. 2A-2G.

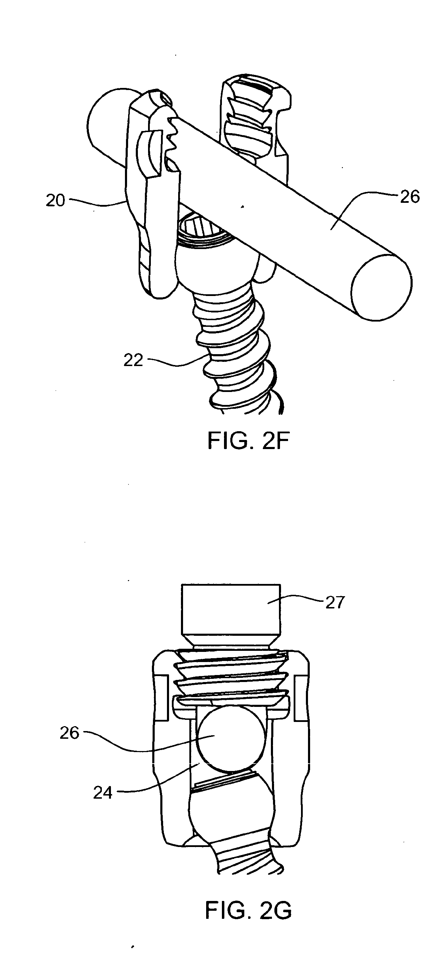

In this embodiment, coupling member 20 is molded around integral head 23 of bone anchor 22. As illustrated in an exemplary embodiment of bone anchor 22 in FIG. 2C, integral head 23 has a rounded or quasi-spherical shape. FIG. 2B illustrates a cutaway view of cavity 21 in coupling member 20 that is formed as a result of molding coupling member 20 to integral head 23 of bone anchor 22. FIGS. 2D and 2E illustrate cutaway and sectional views, respectively, of coupling member 20 molded around the integral head of bone anchor 22. As shown in these figures, coupling member 20 may pivot about the integral head of bone anchor 22. FIGS. 2A, 2B, and 2D also illustrate interface 24 of coupling member 20 that provides an interface to a stabilization member, and threads 25 that are formed in molded coupling member 20. In this embodiment, interface 24 to the stabilization member is integrally formed in molded coupling member 20. As illustrated in the example of FIG. 2A, interface 24 may comprise two spaced apart U-shaped surfaces. FIG. 2F illustrates a cutaway view of coupling member 20 with stabilization member 26 being positioned in interface 24 of coupling member 20. FIG. 2G illustrates a cross-sectional view of this embodiment of the bone anchor system assembled. As illustrated in FIG. 2G, stabilization member 26 is held in coupling member 20 by retaining member 27. In the example shown in FIG. 2G, retaining member 27 comprises a setscrew that engages threads 25.

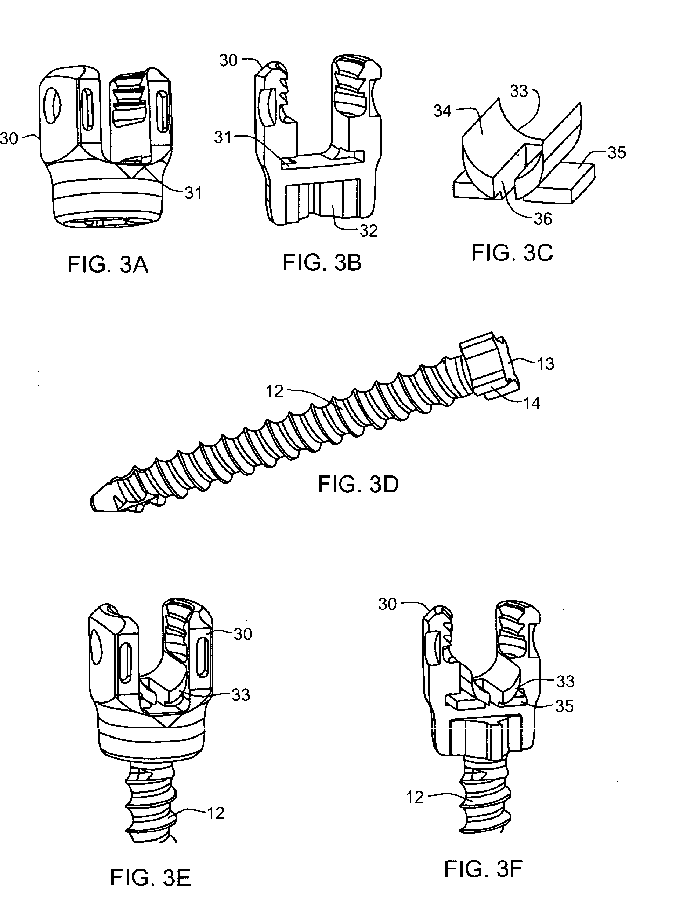

FIGS. 3A-3I illustrate several views of an embodiment of a bone anchor system, in accordance with an aspect of the present invention, wherein a coupling member, which is molded to the head of a bone anchor, includes an inserted interface piece comprising a deformable material. In this embodiment, the interface piece provides an interface to a stabilization member. As illustrated in FIG. 3C, interface piece 33 comprises interface surface 34, at least one tab 35, and collapsible cutout 36. Coupling member 30 also comprises at least one groove 31 as illustrated in FIGS. 3A and 3B. Tabs 35 of interface piece 33 are configured to be retained in a respective groove 31 of coupling member 30 to facilitate interface piece 33 being held in coupling member 30 as illustrated in FIG. 3F. In one embodiment, interface piece 33 may be inserted into coupling member 30 by squeezing the sides of the interface piece to cause collapsible cutout 36 to be compressed, thereby allowing tabs 35 to be inserted into grooves 31 of the interface piece. In this example, interface piece 33 springs back when released, akin to a compression spring, and tabs 35 are retained in grooves 31 to hold interface piece 33 in coupling member 30.

In the embodiments illustrated in FIGS. 3E-3I, coupling member 30 is molded to integral head 13 of bone anchor 12 in a fixed orientation. As illustrated in an exemplary embodiment of bone anchor 12 in FIG. 1C, integral head 13 comprises one or more features 14 that facilitate holding coupling member 30 to integral head 13 of bone anchor 12. FIG. 3A and the cutaway view of FIG. 3B illustrate cavity 32 in coupling member 30 that is formed as a result of molding coupling member 30 to integral head 13 of bone anchor 12.

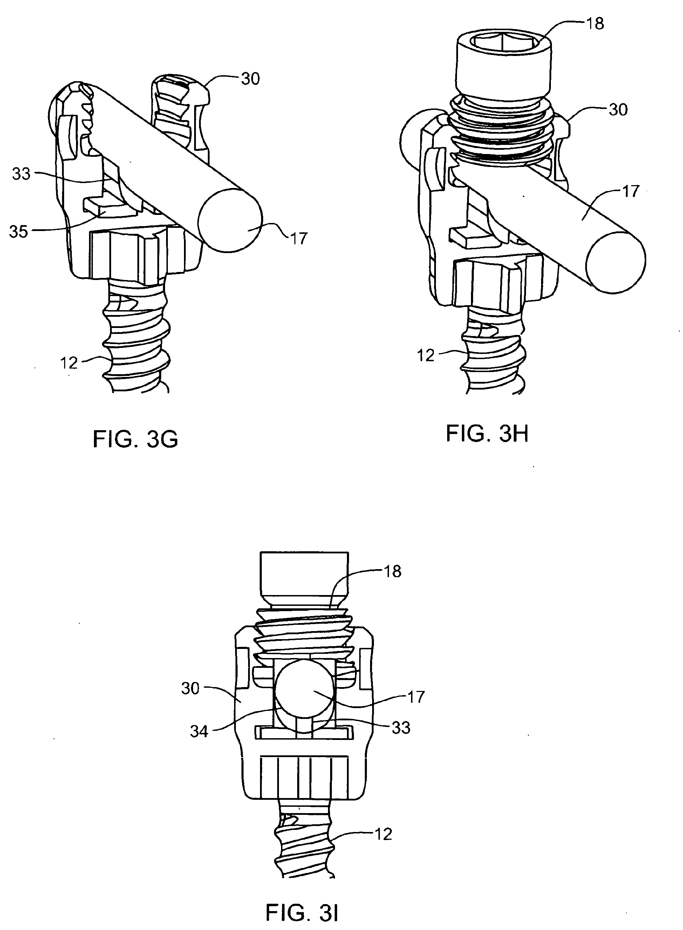

In the example of FIG. 3C, interface surface 34 of interface piece 33 is U-shaped to conform to stabilization member 17, which has a cylindrical shape in the example illustrated in FIG. 3G. As shown in FIGS. 3H and 3I, retaining member 18 engages the threads of coupling member 30. When retaining member 18 is advanced to firmly engage stabilization member 17, stabilization member 17 engages interface surface 34 of interface piece 33 and is held in coupling member 30, as illustrated in the view of FIG 3H and the sectional view of FIG. 3I.

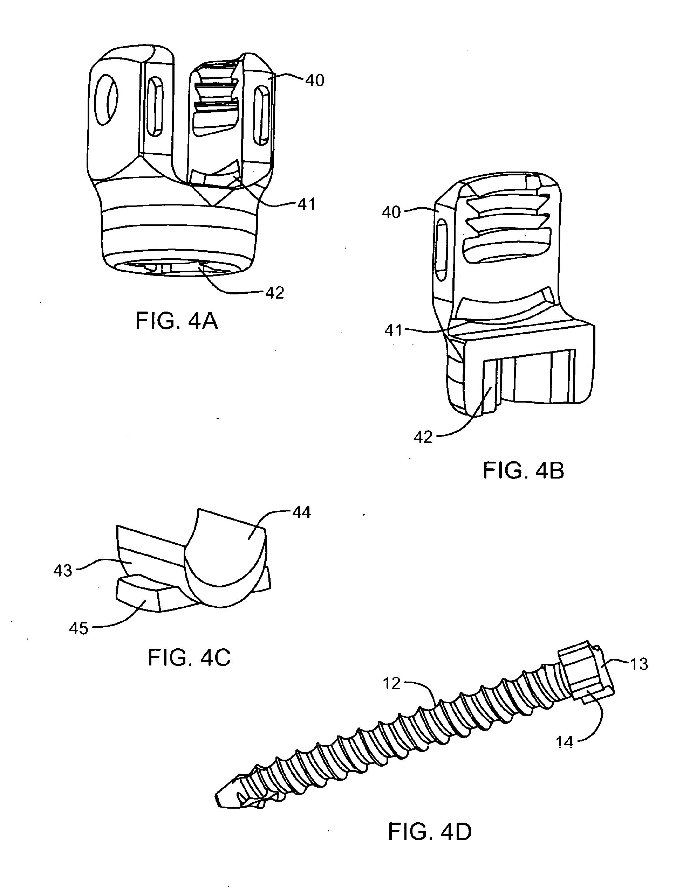

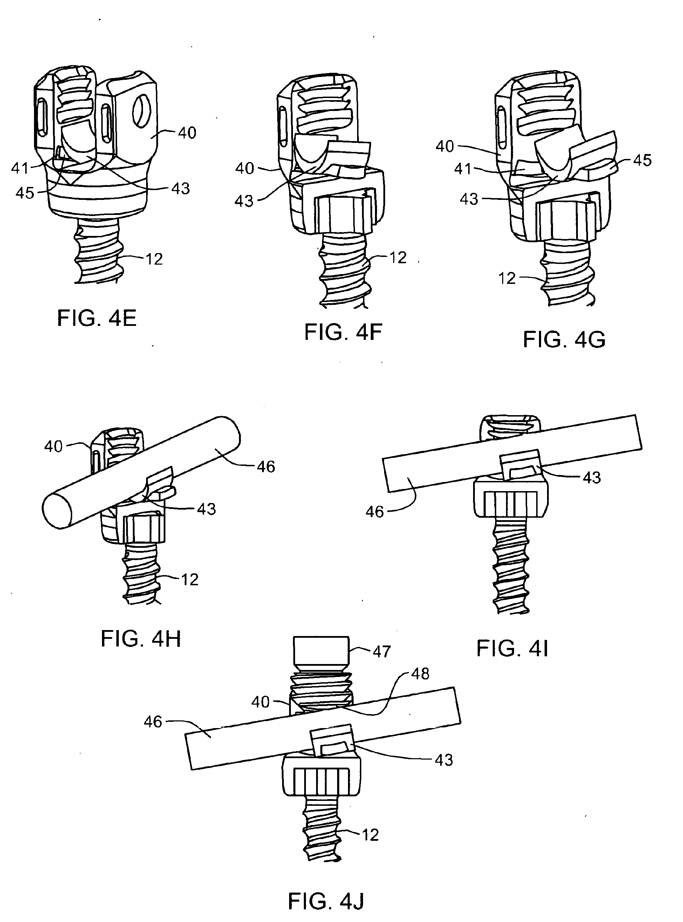

FIGS. 4A-4J illustrate several views of an embodiment of a bone anchor system wherein an inserted interface piece, which comprises a deformable material and provides an interface to a stabilization member, may slide along an arc determined by a pair of groves in a coupling member, which is molded to the head of a bone anchor, in accordance with an aspect of the present invention.

As illustrated in FIG. 4A and the cutaway view of FIG. 4B, coupling member 40 has two elliptical-arc-shaped grooves 41 and a cavity 42 formed by molding coupling member 40 to bone anchor 12. Bone anchor 12 comprises integral head 13 having at least one feature 14 to facilitate holding the molded coupling member to the head of the bone anchor. As illustrated in FIG. 4C, interface piece 43 comprises interface surface 44 for interfacing to a stabilization member and at least one curved tab 45. Curved tabs 45 of interface piece 43 are configured to be retained in a respective groove 41 of coupling member 40 to facilitate interface piece 43 being held in coupling member 40 as illustrated in FIG. 4E and cutaway views 4F and 4G, and curved tabs 45 are contoured such that interface piece 43 may slide along an arc-shaped path defined by clliptical-arc-shaped grooves 41 of coupling member 40.

Interface piece 43 comprises a deformable material. This deformable material may comprise a hard plastic material such a polyetheretherketone (PEEK) material, for example. In one embodiment, interface piece 43 may be inserted into coupling member 40 by squeezing the sides of the interface piece to cause the interface piece to be elastically compressed or deformed to facilitate inserting curved tabs 45 into grooves 41 of coupling member 40. In this example, interface piece 43 springs back when released and curved tabs 45 arc retained in grooves 41 to hold interface piece 43 in coupling member 40. In another embodiment, interface piece 43 may be inserted into coupling member 40 by positioning the corners of curved tabs 45 in respective grooves 41 and twisting interface piece 43 to force curved tabs 45 into in grooves 41.

As shown in the cutaway view of FIG. 4H, stabilization member 46 rests on interface surface 44 of interface piece 43 when the stabilization member is placed in coupling member 40. Stabilization member 46 may be translated along an arc-shaped path as interface piece 43 slides within elliptical-arc-shaped grooves 41 of coupling member 40 as illustrated in the cutaway view of FIG. 4I. This translation of the stabilization member and interface piece together may facilitate proper sagittal alignment of the stabilization member where the bone anchor system embodiment of FIGS. 4A-4J is utilized in a spinal application, for example.

As illustrated in the cutaway view of FIG. 4J, retaining member 47 engages the threads of coupling member 40 to hold stabilization member 46 in coupling member 40 when retaining member 47 is suitably tightened. In addition, in the embodiment illustrated in FIGS. 4A-4J, retaining member 47 may also hold stabilization member 46 and interface piece 43 in a desired position by exerting a retaining force on stabilization member 46. Coupling member 40 is configured to transmit at least a portion of this retaining force to interface piece 43 via stabilization member 46. The portion of the retaining force transmitted to interface piece 43 forces the tabs of interface piece 43 to engage the surfaces of grooves 41 of the coupling member to hold stabilization member 46 and interface piece 43 in a desired position. Retaining member 47 may comprise a setscrew comprising convex tip 48 for engaging stabilization member 46, as illustrated in the embodiment of FIG. 4J for example. In another embodiment, convex tip 48 of the setscrew comprises a hemispherical tip.

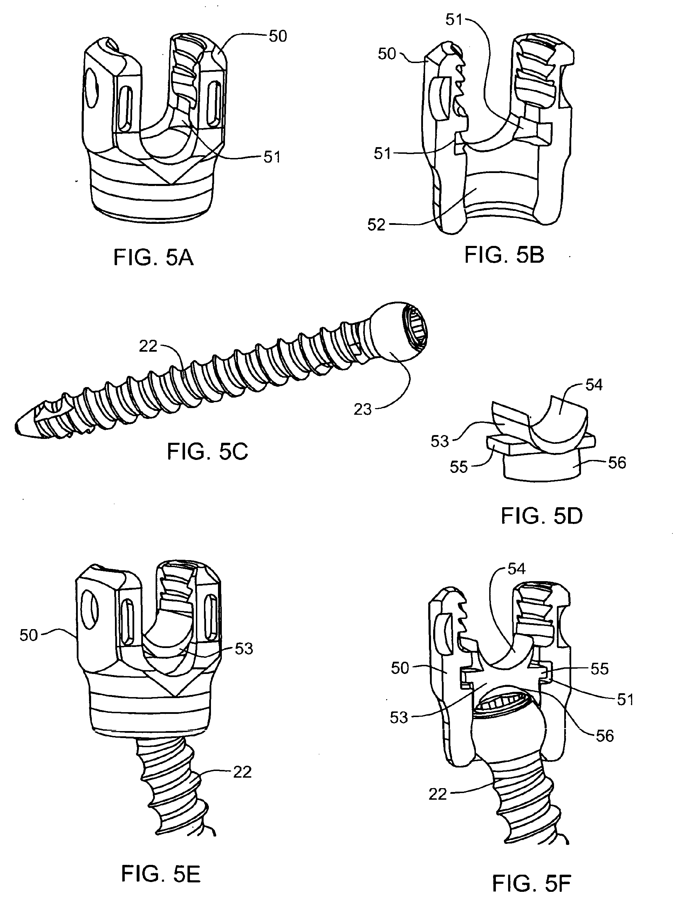

FIGS. 5A-5J illustrate several views of another embodiment of a bone anchor system in which a coupling member and an inserted interface piece, which comprises a deformable material, are configured to arrest the pivoting of the coupling member about the head of a bone anchor when a retaining member engages the coupling member. In this embodiment, coupling member 50 is molded around integral head 23 of bone anchor 22. As illustrated in an exemplary embodiment of bone anchor 22 in FIG. 5C, integral head 23 has a rounded or quasi-spherical shape. FIG. 5B illustrates a cutaway view of cavity 52 in coupling member 50 that is formed as a result of molding coupling member 50 to integral head 23 of bone anchor 22. Cavity 52 of coupling member 50 and integral head 23 of bone anchor 22 arc configured to facilitate moving the molded coupling member pivotally about the head of a bone anchor 22. FIG. 5E and the cutaway view of FIG. 5F illustrate coupling member 50 molded around the integral head of bone anchor 22.

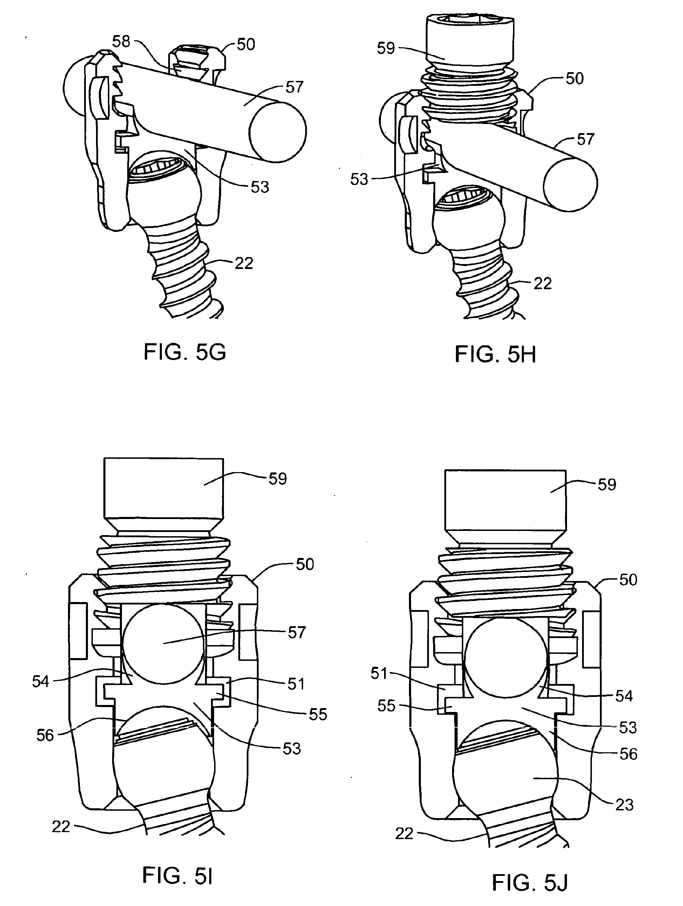

As illustrated in FIGS. 5A, 5B, arid 5F, coupling member 50 has two grooves 51 for receiving interface piece 53, which provides an interface to stabilization member 57, as illustrated in the cutaway view of FIG. 5G, for example. As illustrated in FIG. 5D, interface piece 53 comprises interface surface 54 for interfacing to a stabilization member and at least one tab 55. In the exemplary embodiment illustrated in FIGS. 5A-5J, interface piece 53 comprises two tabs 55. Tabs 55 of interface piece 53 are configured to be retained in a respective groove 51 of coupling member 50 to facilitate interface piece 53 being held in coupling member 50 as illustrated in FIGS. 5E-5J. In addition, interface piece 53 further comprises bone-anchor interface 56 for interfacing to integral head 23 of bone anchor 22. Bone-anchor interface 56 comprises a convex surface for engaging integral head 23 of bone anchor 22, as illustrated in FIG. 5F.

Interface piece 53 may be inserted into coupling member 50 with each of tabs 55 retained in a respective groove 51 of the coupling member, as shown in the cutaway view FIG. 5F. Tabs 55 of interface piece 53 and grooves 51 of coupling member 50 are configured to facilitate sliding interface piece 53 toward cavity 52 of the coupling member to engage integral head 23 of bone anchor 22 as illustrated in FIGS. 5F-5J. This configuration of tabs 55 and grooves 51 facilitates positioning interface piece 53 to arrest the pivoting of coupling member 50 about the integral head of the bone anchor.

In one embodiment, interface piece 53 comprises a deformable material. This deformable material may comprise a hard plastic material such as a polyetheretherketone (PEEK) material, for example. Interface surface 54 of interface piece 53 may be contoured to the shape of the stabilization member, or the interface surface may deform to conform to a shape of the stabilization member along an area of contact between the interface piece and the stabilization member in response to a retaining force applied to the stabilization member. In another embodiment, interface surface 54 of interface piece 53 comprises a deformable material, while another portion of interface piece 53 comprises another biocompatible material such as stainless steel or titanium. For instance, interface surface 54 may comprise a PEEK material or other hard plastic material.

As shown in FIG. 5G, stabilization member 57 rests on interface surface 54 of interface piece 53 when the stabilization member is placed in coupling member 50, and coupling member 50 further comprises threads 58 (FIG. 5G) for engaging retaining member 59 that holds stabilization member 57 in coupling member 50, as illustrated in FIG. 5H. Retaining member 59 comprises a setscrew in one example of the embodiment illustrated in FIGS. 5A-5J. The cross sectional views of FIGS. 5I and 5J illustrate the operation of this embodiment as the retaining member is 59 is tightened. FIG. 5I illustrates retaining member 59 partially engaged in the threads of coupling member 50. With the retaining member partially engaged in the coupling member, bone-anchor interface 56 of interface piece 53 may not forcibly engage integral head 23 of bone anchor 22. In this state, coupling member 50 may pivot about bone anchor 22, or, alternatively, bone anchor 22 may pivot within coupling 50. As illustrated in FIG. 5I, interface piece 53 may be moved further toward integral head 23 of bone anchor 22 because, with the components of this bone anchor system embodiment positioned as shown in FIG. 5I, there is a gap between tabs 55 of interface piece 53 and the ends of grooves 51 of coupling member 50.

As retaining member 59 is advanced to more fully engage threads 58 of coupling member 50, retaining member 59 forces interface piece 53 to move further toward integral head 23 of bone anchor 22. FIG. 5J illustrates a cross sectional view of an embodiment of the bone anchor system, wherein retaining member 59 applies a retaining force to stabilization member 57. Coupling member 50 is configured to facilitate the transmission of at least a portion of the retaining force to interface piece 53 via stabilization member 57 to cause bone-anchor interface 56 of interface piece 53 to forcibly engage the integral head of the bone anchor. By utilizing this aspect of the embodiment illustrated in FIGS. 5A-5J, the retaining member may be used to arrest the pivoting of the coupling member about the integral head of the bone anchor and, thereby, hold the coupling member in a desired pivotal or rotational orientation with respect to the head of the bone anchor. This feature facilitates establishing the required orientation of the stabilization member relative to the bone anchor in order to effect the proper alignment of the subject's bones for the desired medical objective.

In one example of an embodiment of a bone anchor system illustrated in FIGS. 5A-5J, coupling member 50 may comprise a deformable material. This deformable material may comprise a hard plastic material. For instance, the hard plastic material may comprise a polyetheretherketone material. In another example of an embodiment illustrated in FIGS. 5A-5J, coupling member 50 may comprise a biocompatible metal such as titanium or stainless steel, and integral head 23 of bone anchor 23 may comprise a high-temperature coating. The high-temperature coating facilitates the formation of a breakable bond between the integral head of the bone anchor and the coupling member molded thereto. Thus, after the coupling member is molded to the integral head of the bone anchor, the bonds formed between the head of the bone anchor and molded cavity of the coupling member may be broken by applying a pivotal or rotational sheering force, for example, to allow the coupling member to pivot about the integral head of the bone anchor

In other embodiments of a bone anchor system comprising an interface piece for providing an interface to a stabilization member, the coupling member may comprise at least one tab or raised guide for engaging a respective groove in the interface piece. Also, in the embodiments of FIGS. 4A-4J and FIGS. 5A-5J, interface piece 43 and interface piece 53, respectively, may have cutouts, which are similar to cutout 36 of interface piece 33 in FIG. 3C, for example, to facilitate insertion of these interface pieces into the coupling members of the respective embodiments. In some embodiments of present invention, the threads, stabilization member interface, or both may be integrally formed in a molded coupling member by a molding process. In other embodiments, the threads or stabilization member interface of a coupling member, or both, may be machined into a molded coupling member. Also, in the embodiments illustrated in FIGS. 3A-3I, FIGS. 4A-4J, and FIGS. 5A-5J, the interface piece may be inserted by forcing apart the tower portions of the coupling member to facilitate insertion of the interface piece into the coupling member, wherein a tower portion of the coupling member comprises a groove or tab for engaging an interface piece.

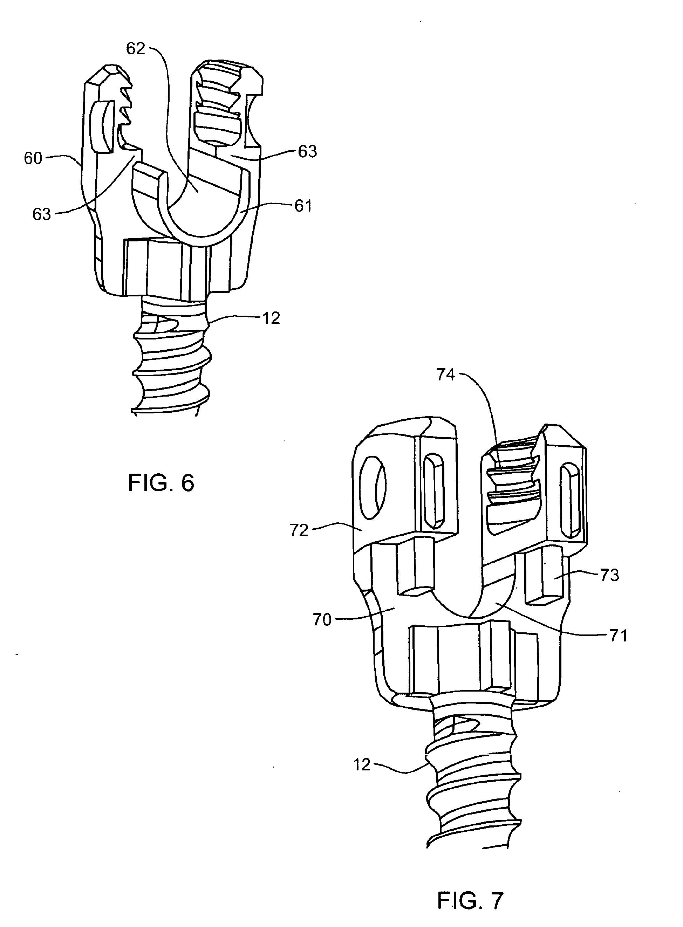

FIG. 6 illustrates a cutaway view of an embodiment of a bone anchor system comprising a coupling member that is adapted to accept an interface piece comprising a deformable material, in accordance with an aspect of the present invention. Coupling member 60 may comprise a biocompatible metal. Examples of suitable biocompatible metal include titanium and stainless steel. Coupling member 60 may be molded around the integral head of bone anchor 12, produced by machining, or produced by a casting process. As illustrated in FIG. 6, coupling member 60 includes interior projecting edges 63 for retaining interface piece 61.

Interface piece 61 provides an interface to a stabilization member and comprises a deformable material. Interface piece 61 further comprises interface surface 62 for contacting a stabilization member. In the example of FIG. 6, interface piece 61 and interface surface 62 are U-shaped for interfacing to a cylindrical stabilization member such as a rod. In one embodiment, interface piece 61 may be molded to conform to the contour of the stabilization member. In another embodiment, interface piece 61 is configured to be sufficiently flexible to facilitate interface piece 61 being fitted to a channel in coupling member 60, wherein the channel is configured to support interface piece 61. The channel and fitted, flexible interface piece cooperatively provide an interface surface that is contoured to the stabilization member. The deformable material may comprise a hard plastic for example. An exemplary hard plastic material comprises a PEEK material.

FIG. 7 illustrates a cutaway view of an embodiment of a bone anchor system wherein a coupling member, which is molded to the head of a bone anchor, engages an insert that is configured to engage a retaining member, in accordance with an aspect of the present invention. In the embodiment illustrated in FIG. 7, coupling member 70 is molded around the integral head of bone anchor 12, wherein the integral head of bone anchor 12 comprises one or more features that facilitate holding coupling member 70 to the integral head of the bone anchor in a fixed orientation.

As illustrated in FIG. 7, coupling member 70 comprises interface 71 that is configured for engaging a stabilization member, such as a rod. In the example shown, interface 71 is integrally formed in coupling member 70. In addition, coupling member 70 is configured to engage posts 73 of threaded member 72. Threaded member 72 further comprises threads 74 that have been integrally formed in threaded member 72. In the example shown in FIG. 7, threads 74 of threaded member 72 may engage a setscrew or other retaining member to hold a stabilization member that may be placed in interface 71 of coupling member 70.

As illustrated in the exemplary embodiment in FIG. 7, interface 71 to the stabilization member may comprise a U-shaped surface where the stabilization member has a cylindrical shape. However, in other embodiments, the interface to the stabilization member may comprise a surface that is contoured to the shape of another stabilization member in the region of contact between the interface and stabilization member. In one embodiment, coupling member 70 comprises a hard plastic material, and threaded member 72 comprises a biocompatible metal. This embodiment advantageously may provide strong, durable, metal threads in the threaded member, while also providing a hard plastic stabilization-member interface which may advantageously distribute forces transmitted to a stabilization member. For example, the hard plastic material may comprise a polyetheretherketone material, and the biocompatible metal may comprise titanium or stainless steel.

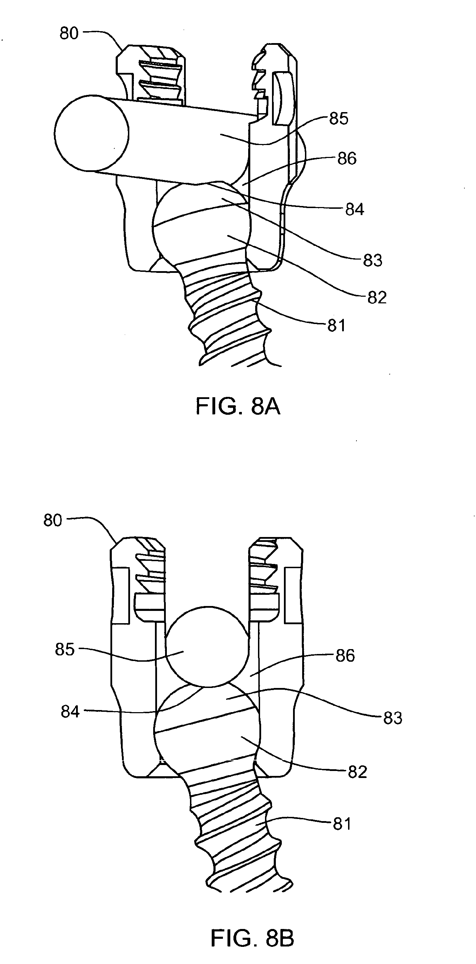

FIGS. 8A and 8B illustrate cutaway and sectional views, respectively, of an embodiment of a bone anchor system wherein the integral head of a bone anchor comprises a molded cap of deformable material, and the molded cap provides an interface to a stabilization member, in accordance with an aspect of the present invention.

In this embodiment, coupling member 80 is molded around integral head 82 of bone anchor 81. Coupling member 80 and integral head 82 of bone anchor 81 are configured to allow coupling member 80 to pivot about integral head 82. As illustrated in the example of FIGS. 8A and 8B, integral head 82 comprises molded cap 83 and has a rounded or quasi-spherical shape. Molded cap 83 of integral head 82 may comprise a deformable material and may be configured to provide an interface to stabilization member 85. Coupling member 80 further comprises interface 86 to stabilization member 85. Interface 86 of coupling member 80 is configured with an opening that facilitates stabilization member 85 engaging molded cap 83 of bone anchor 81 when stabilization member 85 is placed in interface 86 of coupling member 80. Coupling member 80 is further configured to engage a retaining member. For example, as illustrated in FIGS. 8A and 8B, coupling member 80 may further comprise threads for engaging a setscrew. In this example, a setscrew (not shown) may engage the threads of coupling member 80 and may apply a retaining force on stabilization member 85. Molded cap 83 of integral head 82 of bone anchor 81 provides an interface to stabilization member 85. FIGS. 8A and 8B illustrate interface surface 84 deforming to conform to the shape of stabilization member 85 along an area of contact between interface surface 84 of the molded cap and stabilization member 85 as the stabilization member engages interface surface 84 of the molded cap. This may occur, for example, if a retaining member applies a retaining force on stabilization member 85, causing the stabilization member to forcibly engage molded cap 83.

Since interface 86 of coupling member 80 is configured to facilitate the transmission of at least a portion of the retaining force to interface surface 84 of molded cap 83 via stabilization member 85, the retaining member may be used to arrest the pivoting of coupling member 80 about the integral head of the bone anchor 81 and, thereby, hold the coupling member in a desired pivotal or rotational orientation with respect to the head of the bone anchor. This capability facilitates establishing the required orientation of the stabilization member relative to the bone anchor in order to effect the proper alignment of the subject's bones for the desired medical objective.

In the embodiment illustrated in FIGS. 8A and 8B, coupling member 80 comprises a deformable material, and molded cap 83 of the integral head of bone anchor 81 also comprises a deformable material. The deformable material may comprise a hard plastic, for example. The hard plastic may comprise a PEEK material, for example. In other instances, the deformable material may comprise a Teflon, nylon, silicone, or polymer material. In further examples, the deformable material may comprise a radiolucent material, a non-conducting material, or a non-magnetic material. Radiolucent, non-conducting, and non-magnetic materials may be advantageous because they are more compatible with commonly used medical diagnostic imaging methods than biocompatible metals.

In another aspect of the present invention, a method of providing a coupling between a stabilization member and bone anchor of a bone anchor system comprises the following steps: molding a coupling member to an integral head of a bone anchor; providing, in the coupling member , an interface to a stabilization member; providing, in the coupling member, a means for engaging a retaining member; and configuring the coupling and the retaining members to cooperatively retain the stabilization member when the stabilization member is placed in the interface of the coupling and the retaining member is engaged in the means for engaging the retaining member. For example, the retaining member may comprise a setscrew, and the means for engaging the retaining member may comprise threads.

In one embodiment of this method, the step of molding a coupling member to the integral head of a bone anchor comprises orienting a mold over the integral head of the bone anchor. With the mold in place, a coupling member may be produced by an injection molding process, which is well-known to one of ordinary skill in the art. Another embodiment of the method may include placing an interface piece in the in mold along with the integral head of the bone anchor and molding a coupling member around both the head of the hone anchor and interface piece. In an embodiment in which the bone anchor comprises a rounded or quasi-spherical head to facilitate pivoting the coupling member about the head of the bone anchor, a pivotal or rotational sheering force may be applied to break the bonds formed between the head of the bone anchor and molded cavity of the coupling member to allow the coupling member to pivot about the integral head of the bone anchor.

Another embodiment of a method of providing a coupling between a stabilization member and bone anchor of a bone anchor system comprises: forming a molded coupling member to an integral head of a bone anchor, wherein the forming comprises a heat staking process; providing, in the molded coupling member, an interface to a stabilization member; providing, in the molded coupling member, a means for engaging a retaining member; and configuring the molded coupling member and the retaining member to cooperatively retain the stabilization member when the stabilization member is placed in the interface of the molded coupling member and the retaining member is engaged in the means for engaging the retaining member. In this embodiment, the molded coupling member comprises a deformable material. The details of forming a deformable component about a metal component, such as the head of a bone anchor, by heat-staking will be appreciated by one of ordinary skill in the art.

It is understood that, in the various embodiments of present invention, the means for engaging a retaining member included in a coupling member is not limited to threads, but may comprise helical protrusions and features for securing the retaining member in the coupling member, for example.

## Claims

1. A method for manufacturing a bone anchor system, the bone anchor system comprising:
a bone anchor (12; 22; 81) having an integral head (13; 23; 82); and
a coupling member (10; 20; 30; 40; 50; 60; 70; 80) for coupling the bone anchor to a stabilization member,
**characterised in that** the coupling member (10; 20; 30; 40; 50; 60; 70; 80) is moulded around the integral head of the bone anchor (13; 23; 82) by an injection moulding process.

2. The method of claim 1, the bone anchor system further comprising a stabilization member (17; 26; 46; 57; 85).

3. The method of claim 1 wherein the integral head of the bone anchor (13; 23; 82) comprises at least one feature to facilitate securing the coupling member (10; 20; 30; 40; 50; 60; 70; 80) to the integral head of the bone anchor (13; 23; 82).

4. The method of claim 1, wherein the coupling member (30; 40; 50; 60; 80) comprises a biocompatible metal, and the coupling member (30; 40; 50; 60; 80) further comprises an interface (33; 43; 53; 51; 83) to the stabilization member (17; 46; 57; 85), the interface (33; 43; 53; 51; 83) comprising a deformable material.

5. The method of claim 1, wherein the integral head of the bone anchor (23; 82) is configured to allow the coupling member (20; 50; 80) to pivot about the integral head of the bone anchor (23; 82).

6. The method of claim 1 , wherein the bone anchor system further comprises a retaining member (18; 59) for holding the stabilization member (17; 26; 46; 57; 85) in the coupling member (10; 20; 30; 40; 50; 60; 70; 80).

7. The method of claim 1 wherein the integral head of the bone anchor (82) comprises an interface to the stabilization member (83), the interface comprising a deformable material.

8. The method of claim 7, wherein the system further comprises a retaining member, the retaining member exerting a retaining force on the stabilization member (85) when the retaining member is engaged in the coupling member (80); and the coupling member (80) being configured to transmit at least a portion of the retaining force to the interface via the stabilization member (85) to cause the stabilization member (85) to engage the interface of the integral head of the bone anchor (83).

9. The method of claim 8, wherein a surface of the interface of the integral head of the bone anchor (83) deforms to conform to a shape of the stabilization member (85) along an area of contact between the interface (83) and the stabilization member (85) as the stabilization member (85) engages the interface (83).

10. The method of claim 7, wherein the integral head of the bone anchor (82) is configured to allow the coupling member (80) to pivot about the integral head of the bone anchor (82).

## Patentansprüche

1. Verfahren zur Herstellung eines Knochenankersystems, wobei das Knochenankersystem folgendes umfasst:
einen Knochenanker (12; 22; 81) mit einem integralen Gewinde (13; 23; 82); und
einem Kopplungselement (10; 20; 30; 40; 50; 60; 70; 80) zum Koppeln des Knochenankers mit einem Stabilisierungselement;
**dadurch gekennzeichnet, dass** das Kopplungselement (10; 20; 30; 40; 50; 60; 70; 80) durch ein Spritzgussverfahren um den integralen Kopf des Knochenankers (13; 23; 82) geformt ist.

2. Verfahren nach Anspruch 1, wobei das Knochenankersystem ferner ein Stabilisierungselement (17; 26; 46; 57; 85) umfasst.

3. Verfahren nach Anspruch 1, wobei der integrale Kopf des Knochenankers (13; 23; 82) mindestens ein Merkmal umfasst, dass das Sichern des Kopplungselements (10; 20; 30; 40; 50; 60; 70; 80) an dem integralen Kopf des Knochenankers (13; 23; 82) ermöglicht.

4. Verfahren nach Anspruch 1, wobei das Kopplungselement (30; 40; 50; 60; 80) ein biokompatibles Metall umfasst, und wobei das Kopplungselement (30; 40; 50; 60; 80) ferner eine Schnittstelle (33; 43; 53; 51; 83) mit dem Stabilisierungselement (17; 46; 57; 85) umfasst, wobei die Schnittstelle (33; 43; 53; 51; 83) ein verformbaresMaterial umfasst.

5. Verfahren nach Anspruch 1, wobei der integrale Kopf des Knochenankers (23; 82) so konfiguriert ist, dass er es ermöglicht, dass sich das Kopplungselement (20; 50; 80) um den integralen Kopf des Knochenankers (23; 82) dreht.

6. Verfahren nach Anspruch 1, wobei das Knochenankersystem ferner ein Halteelement (18; 59) zum Halten des Stabilisierungselements (17; 26; 46; 57; 85) in dem Kopplungselement (10; 20; 30; 40; 50; 60; 70; 80) umfasst.

7. Verfahren nach Anspruch 1, wobei der integrale Kopf des Knochenankers (82) eine Schnittstelle mit dem Stabilisierungselement (83) umfasst, wobei die Schnittstelle ein verformbares Material umfasst.

8. Verfahren nach Anspruch 7, wobei das System ferner ein Halteelement umfasst, wobei das Halteelement eine Haltekraft auf das Stabilisierungselement (85) ausübt, wenn das Halteelement in dem Kopplungselement (80) eingreift; und wobei das Kopplungselement (80) so konfiguriert ist, dass es zumindest einen Teil der Haltekraft über das Stabilisierungselement (85) auf die Schnittstelle überträgt, um zu bewirken, dass das Stabilisierungselement (85) mit der Schnittstelle des integralen Kopfs des Knochenankers (83) eingreift.

9. Verfahren nach Anspruch 8, wobei sich eine Oberfläche der Schnittstelle des integralen Kopfs des Knochenankers (83) so verformt, dass sie mit einer Form des Stabilisierungselements (85) entlang einer Berührungsfläche zwischender Schnittstelle (83) und dem Stabilisierungselement (85) zusammenpasst, wenn das Stabilisierungselement (85) mit der Schnittstelle (83) eingreift.

10. Verfahren nach Anspruch 7, wobei der integrale Kopf des Knochenankers (82) so konfiguriert ist, dass er es ermöglicht, dass sich das Kopplungselement (80) um einen integralen Kopf des Knochenankers (82) dreht.

## Revendications

1. Procédé de fabrication d'un système d'ancrage osseux, le système d'ancrage osseux comprenant :
un ancrage osseux (12 ; 22 ; 81) ayant une tête intégrale (13 ; 23 ; 82) ; et
un élément de couplage (10 ; 20 ; 30 ; 40 ; 50 ; 60 ; 70 ; 80) pour coupler l'ancrage osseux à un élément stabilisant,
**caractérisé en ce que** l'élément de couplage (10 ; 20 ; 30 ; 40 ; 50 ; 60 ; 70 ; 80) est moulé autour de la tête intégrale de l'ancrage osseux (13 ; 23 ; 82) par un procédé de moulage par injection.

2. Procédé selon la revendication1, le système d'ancrage osseux comprenant en outre un élément stabilisant (17 ; 26 ; 46 ; 57 ; 85).

3. Procédé selon la revendication1, dans lequel la tête intégrale de l'ancrage osseux (13 ; 23 ; 82) comprend au moins une fonction pour faciliter la fixation de l'élément de couplage (10 ; 20 ; 30 ; 40 ; 50 ; 60 ; 70 ; 80) à la tête intégrale de l'ancrage osseux (13 ; 23 ; 82).

4. Procédé selon la revendication1, dans lequel l'élément de couplage (30 ; 40 ; 50 ; 60 ; 80) comprend un métal biocompatible, et l'élément de couplage (30 ; 40 ; 50 ; 60 ; 80) comprend en outre une interface (33 ; 43 ; 53 ; 51 ; 83) vers l'élément stabilisant (17 ; 46 ; 57 ; 85), l'interface (33 ; 43 ; 53 ; 51 ; 83) comprenant un matériau déformable.

5. Procédé selon la revendication1, dans lequel la tête intégrale de l'ancrage osseux (23 ; 82) est configurée pour permettre à l'élément de couplage (20 ; 50 ; 80) de pivoter autour de la tête intégrale de l'ancrage osseux (23 ; 82).

6. Procédé selon la revendication1, dans lequel le système d'ancrage osseux comprend en outre un élément de retenue (18 ; 59) pour tenir l'élément stabilisant (17 ; 26 ; 46 ; 57 ; 85) dans l'élément de couplage (10 ; 20 ; 30 ; 40 ; 50 ; 60 ; 70 ; 80).

7. Procédé selon la revendication1, dans lequel la tête intégrale de l'ancrage osseux (82) comprend une interface vers l'élément stabilisant (83), l'interface comprenant un matériau déformable.

8. Procédé selon la revendication7, dans lequel le système comprend en outre un élément de retenue, l'élément de retenue exerçant une force de retenue sur l'élément stabilisant (85) lorsque l'élément de retenue vient en prise dans l'élément de couplage (80) ; et l'élément de couplage (80) étant configuré pour transmettre au moins une partie de la force de retenue à l'interface via l'élément stabilisant (85) pour amener l'élément stabilisant (85) à venir en prise avec l'interface de la tête intégrale de l'ancrage osseux (83).

9. Procédé selon la revendication8, dans lequel une surface de l'interface de la tête intégrale de l'ancrage osseux (83) se déforme pour prendre la forme de l'élément stabilisant (85) le long d'une surface de contact entre l'interface (83) et l'élément stabilisant (85) lorsque l'élément stabilisant (85) vient en prise avec l'interface (83).

10. Procédé selon la revendication7, dans lequel la tête intégrale de l'ancrage osseux (82) est configurée pour permettre à l'élément de couplage (80) de pivoter autour de la tête intégrale de l'ancrage osseux (82).
